(19) Europäisches Patentamt
European Patent Office
Office européen des brevets

(11) Veröffentlichungsnummer : **0 132 811**
**B1**

(12) # EUROPÄISCHE PATENTSCHRIFT

(45) Veröffentlichungstag der Patentschrift : **28.09.88**

(51) Int. Cl.⁴ : **C 07 D207/263**, C 07 D401/06, A 61 K 31/40, A 61 K 31/44

(21) Anmeldenummer : **84108642.4**

(22) Anmeldetag : **21.07.84**

(54) **In 1-Stellung substituierte 4-Hydroxymethyl-pyrrolidinone, Verfahren zu ihrer Herstellung, pharmazeutische Zusammensetzungen und Zwischenprodukte.**

(30) Priorität : **25.07.83 DE 3326724**

(43) Veröffentlichungstag der Anmeldung : **13.02.85 Patentblatt 85/07**

(45) Bekanntmachung des Hinweises auf die Patenterteilung : **28.09.88 Patentblatt 88/39**

(84) Benannte Vertragsstaaten : **AT BE CH DE FR GB IT LI LU NL SE**

(56) Entgegenhaltungen :
**US-A- 3 459 738**
**CHEMICAL ABSTRACTS, Band 98, Nr. 19, 9. Mai 1983, Columbus, Ohio, USA LABOUTA, IBRAHIM M. et al. "Synthesis of some cyclic amino acids structurally related to the GABA analog homo-B-proline" Seite 551, Spalte 1, Zusammenfassung-Nr. 161 134k**
**CHEMICAL ABSTRACTS, Band 96, Nr. 3, 1. Jänner 1982, Columbus, Ohio, USA MOEHRIE, H. et al. "Oxidative cleavage of 2-(pyrrolidinomethyl)-pyridine 1-oxide with neighboring recombination" Seite 434, Spalte 2, Zusammenfassung-Nr. 19 922f**
**CHEMICAL ABSTRACTS, Band 92, Nr. 17, 28. April 1980, Columbus, Ohio, USA TAKAHATA, HIROKI et al. "N-Alkylation of lactams with phase transfer catalyst" Seite 573, Spalte 1, Zusammenfassung-Nr. 146 577q**
**CHEMICAL ABSTRACTS, Band 86, Nr. 19, 9. Mai 1977, Columbus, Ohio, USA SATTLER, H.J. et al. "Effects of partially cyclic and ring-methylated nikethamid analogs" Seite 17, Spalte 1, Zusammenfassung-Nr. 133 354r**
**Die Akte enthält technische Angaben, die nach dem Eingang der Anmeldung eingereicht wurden und die nicht in dieser Patentschrift enthalten sind.**

(73) Patentinhaber : **BOEHRINGER INGELHEIM KG**
**Postfach 200**
**D-6507 Ingelheim am Rhein (DE)**
**BE CH DE FR IT LI LU NL SE AT**
**BOEHRINGER INGELHEIM INTERNATIONAL G.M.B.H.**
**Postfach 20**
**D-6507 Ingelheim am Rhein (DE)**
**GB**

(72) Erfinder : **Weber, Karl-Heinz, Dr.**
**Kaiser-Karl-Strasse 11**
**D-6535 Gau-Algesheim (DE)**
Erfinder : **Walther, Gerhard, Dr.**
**Pfarrer-Heberer-Strasse 37**
**D-653 Bingen/Rhein (DE)**
Erfinder : **Schneider, Claus, Dr.**
**Albrecht-Dürer-Strasse 19**
**D-6507 Ingelheim am Rhein (DE)**
Erfinder : **Hinzen, Dieter, Prof. Dr.**
**Stromberger Strasse 36**
**D-653 Bingen/Rhein (DE)**
Erfinder : **Kuhn, Franz Josef, Dr.**
**Beethovenstrasse 11**
**D-6535 Gau-Algesheim (DE)**
Erfinder : **Lehr, Erich, Dr.**
**In der Toffel 5**
**D-6531 Waldalgesheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 132 811 B1**

Jouve, 18, rue St-Denis, 75001 Paris, France

**Beschreibung**

Die Erfindung betrifft neue in 1-Stellung substituierte 4-Hydroxymethyl-pyrrolidinone, ein Verfahren zu ihrer Herstellung, ihre Anwendung in pharmazeutischen Präparaten und die als Zwischenprodukte verwendeten neuen Säuren und Ester. Die neuen Verbindungen haben sich im Tierversuch als wirksam bei der Verminderung beziehungsweise Aufhebung von Zustandsformen eingeschränkter cerebraler Leistungsfähigkeit erwiesen.

Als strukturell ähnlich gebaute Nootropica sind das 1-Carbamoylmethyl-pyrrolidin-2-on (Pirazetam), das 1-(p-Methoxybenzoyl)-pyrrolidin-2-on (Anirazetam) und das 1-Carbamoylmethyl-4-hydroxy-pyrrolidin-2-on (Oxirazetam) bereits in der Literatur beschrieben, siehe B. J. R. Nicolaus, Drug Development Res. 2, 464 (1982), P. L. Paytasch, J. Amer. Chem. Soc. 72, 1415 (1950).

Überraschenderweise wurde nun gefunden, daß bereits eine relativ geringfügige Veränderung im Molekül die Wirkung der bekannten Nootropica ganz wesentlich verbessert.

Gegenstand der Erfindung sind neue in 1-Stellung substituierte 4-Hydroxymethyl-pyrrolidinone der allgemeinen Formel

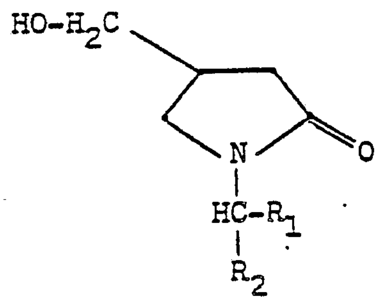

(I)

in der

$R_1$ Wasserstoff oder Methyl und

$R_2$ einen gegebenenfalls durch Alkyl mit 1-2 Kohlenstoffatomen, Alkoxy mit 1-2 Kohlenstoffatomen, Fluor, Chlor, Brom, Trifluormethyl, Nitro, Benzyloxy oder Hydroxy ein-, zwei- oder dreifach substituierten Phenylrest oder einen Pyridylrest bedeutet.

Hervorgehoben seien solche Verbindungen der allgemeinen Formel I, die einen unsubstituierten oder einen ein-, zwei- oder dreifach durch Methoxy substituierten Phenylring tragen.

Die neuen Verbindungen besitzen ein Asymmetriezentrum und liegen daher als Racemate vor. Diese Racemate können in üblicher Weise, z. B. durch Veresterung mit optisch aktiven Säuren, in die entsprechenden optisch aktiven Ester überführt werden, die anschließend zu den optisch aktiven Formen der Endprodukte verseift werden.

Gegenstand der Erfindung sind ferner die als Zwischenprodukte verwendeten neuen Säuren und Ester der allgemeinen Formel

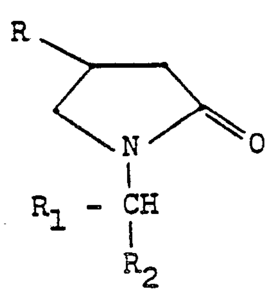

(II)

in der

$R_1$ Wasserstoff oder Methyl

$R_2$ eine durch Alkyl mit 1-2 Kohlenstoffatomen, Alkoxy mit 1-2 Kohlenstoffatomen, Fluor, Chlor, Brom, Trifluormethyl, Nitro, Benzyloxy oder Hydroxy ein-, zwei- oder dreifach substituierten Phenylrest oder einen Pyridylrest und

R einen der Reste —COOH und —COO—$R_3$ mit $R_3$ = Alkyl mit 1 bis 2 Kohlenstoffatomen bedeuten.

Die Erfindung betrifft ferner ein Verfahren zur Herstellung der neuen Verbindungen der allgemeinen Formel I, welches darin besteht, daß man einen Pyrrolidinon-4-carbonsäureester der allgemeinen Formel

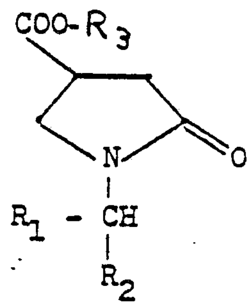

(IIa)

in der

$R_1$ und $R_2$ die oben angeführte Bedeutung besitzen, mit einem komplexen Alkali-Borhydrid selektiv reduziert.

Die selektive Reduktion wird mit komplexen Alkali-Borhydriden unter Erhalt der Amidfunktion im Ring durchgeführt. Als Lösungsmittel für die Reduktion können niedere Alkohole wie Methanol oder Butanol, gegebenenfalls unter Wasserzusatz, verwendet werden. Die Reaktionstemperaturen liegen zwischen — 5 °C und dem Siedepunkt des Alkohols.

Zwecks Herstellung einer Verbindung der allgemeinen Formel I, worin $R_2$ einen hydroxysubstituierten Phenylrest bedeutet und $R_1$ die oben genannte Bedeutung hat, wird eine Verbindung der allgemeinen Formel I, worin $R_2$ einen entsprechenden benzyloxysubstituierten Phenylrest bedeutet, katalytisch hydriert.

Die Hydrierung erfolgt zweckmäßigerweise in Gegenwart eines organischen Lösungsmittels, z. B. Methanol, und eines Hydrierungskatalysators, z. B. Pd/C.

Die als Zwischenprodukte verwendeten Säuren und Ester der allgemeinen Formel II können aus äquimolaren Mengen Itaconsäure und den entsprechenden Aminen mit gegebenenfalls anschließender Veresterung gemäß dem folgenden Schema erhalten werden :

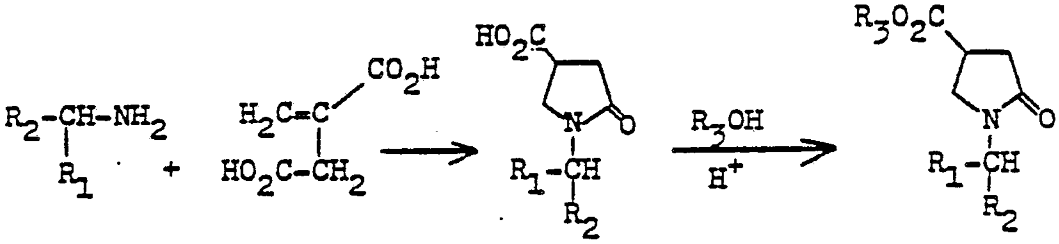


$R_3$ = Alkyl mit 1 bis 2 Kohlenstoffatomen.

In manchen Fällen ist es ferner möglich, von einem Itaconsäureester der allgemeinen Formel

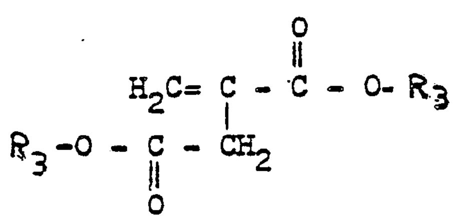

auszugehen und diesen direkt mit dem gewünschten Amin zum Ester II umzusetzen.

Die Cycloaddition zur Pyrrolidinoncarbonsäure kann in siedendem Wasser oder ohne Lösungsmittel, zweckmäßig in einer Inertgasatmosphäre z. B. $N_2$ bei Temperaturen von 100-150 °C erfolgen.

Nach dem oben beschriebenen Verfahren können beispielsweise die folgenden Endprodukte erhalten werden :

1-Benzyl-4-hydroxymethyl-pyrrolidin-2-on
1-(2-Methoxybenzyl)-4-hydroxymethyl-pyrrolidin-2-on
1-(3-Methoxybenzyl)-4-hydroxymethyl-pyrrolidin-2-on
1-(4-Methoxybenzyl)-4-hydroxymethyl-pyrrolidin-2-on
1-(3,4-Dimethoxybenzyl)-4-hydroxymethyl-pyrrolidin-2-on
1-(3,4,5-Trimethoxybenzyl)-4-hydroxymethyl-pyrrolidin-2-on
1-(4-Methylbenzyl)-4-Hydroxymethyl-pyrrolidin-2-on
1-(4-Chlorbenzyl)-4-hydroxymethyl-pyrrolidin-2-on
1-(2-Chlorbenzyl)-4-hydroxymethyl-pyrrolidin-2-on

1-(4-Fluorbenzyl)-4-hydroxymethyl-pyrrolidin-2-on
1-(3-Trifluormethylbenzyl)-4-hydroxymethyl-pyrrolidin-2-on
1-(3-Hydroxy-4-methoxybenzyl)-4-hydroxymethyl-pyrrolidin-2-on
1-(3-Methoxy-4-hydroxybenzyl)-4-hydroxymethyl-pyrrolidin-2-on
1-(2-Methyl-4-chlorbenzyl)-4-hydroxymethyl-pyrrolidin-2-on
1-(α-Methylbenzyl)-4-hydroxymethyl-pyrrolidin-2-on
1-(α-Methyl-4-methoxybenzyl)-4-hydroxymethyl-pyrrolidin-2-on
1-Pyridyl-(2)-methyl-4-hydroxymethyl-pyrrolidin-2-on
1-Pyridyl-(3)-methyl-4-hydroxymethyl-pyrrolidin-2-on
1-Pyridyl-(4)-methyl-4-hydroxymethyl-pyrrolidin-2-on
1-(4-Hydroxybenzyl)-4-hydroxymethyl-pyrrolidin-2-on

Die neuen Pyrrolidinon-Derivate wurden in Tierexperimenten bezüglich ihrer Wirksamkeit untersucht, Zustandsformen eingeschränkter cerebraler Leistungsfähigkeit aufzuheben beziehungsweise zu mindern.

Die Verbindungen weisen in orientierenden Verträglichkeitsuntersuchungen an der Maus in Dosierungen bis zu 2 g/kg (einmalige orale Applikation) keine akute Toxizität (14 Tage Nachbeobachtung) auf. Sie zeigen tierexperimentell ausgezeichnete Wirkungen auf spontane kognitive Leistungen, wie experimentell eingeschränkte Lern- und Gedächtnisvorgänge. In Versuchen mit Einschränkung des Kurzzeitgedächtnisses beziehungsweise Behinderung des Übergangs von Inhalten des Kurzzeitin das Langzeitgedächtnis durch Gabe eines muscarinen cholinergen Antagonisten (Scopolamin 0,6 mg/kg i. p. ; siehe auch Psychopharmacology 78, 104-111 (1982)), sind die Verbindungen in der Lage, dieser pharmakologisch induzierten cerebralen Insuffizienz entgegenzuwirken, beziehungsweise sie aufzuheben.

Die Lernfähigkeit von Ratten in einer aktiven Vermeidedressur (J. pharmacol. Methods, 8, 255-263 (1983) wird ebenso verbessert wie ihre spontane Habituation beziehungsweise explorierende Orientierungsaktivität in einer neuen Umgebung.

Die neuen Pyrrolidinon-Derivate wurden in ihrer Wirksamkeit mit andersstrukturierten Pyrrolidinonen verglichen, welche im Rahmen der cerebralen Insuffiziens beziehungsweise des hirnorganischen Psychodroms, der posttraumatischen und alkoholischen Hirnschädigung usw., in der Humanmedizin bereits als Arzneimittel Anwendung finden (Pirazetam) beziehungsweise zur Zeit klinisch erprobt werden (Anriazetam).

Die neuen Verbindungen sind sowohl bezüglich der wirksamen Dosis als auch der im Tierexperiment erzielten Leistungsverbesserung den genannten Substanzen deutlich überlegen.

Die neuen Verbindungen können allein oder in Kombination mit anderen erfindungsgemäßen Wirkstoffen, gegebenenfalls auch in Kombination mit weiteren pharmakologisch aktiven Wirkstoffen, z. B. weiteren Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver. Entsprechende tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstofe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z. B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z. B. unter Zusatz von Konservierungsmitteln, wie p-Hydroxybenzoaten, oder Stabilisatoren, wie Alkalisalzen der Äthylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die nachfolgenden Beispiele illustrieren die vorliegende Erfindung, ohne sie jedoch in ihrem Umfang zu beschränken :

Beispiel 1

1-(3,4-Dimethoxybenzyl)-4-hydroxymethyl-pyrrolidin-2-on

8,0 g 1-(3,4-Dimethoxybenzyl)-4-äthoxycarbonyl-pyrrolidin-2-on werden in 100 ml Methanol gelöst. Unter mechanischem Rühren tropft man während 20 Minuten bei 0-10 °C eine Lösung von 2,8 g Natriumborhydrid in 30 ml Wasser zu und rührt weitere 5 Stunden bei 0-10 °C. Anschließend wird das überschüssige Borhydrid vorsichtig durch Zugabe von Eisessig bis zu einem pH von 5 und bis zum Aufhören der Gasentwicklung versetzt und das Methanol im Vakuum abdestilliert. Man setzt etwas Wasser zu und nimmt das Reaktionsprodukt in Methylenchlorid auf. Die Methylenchloridphase wird anschließend gewaschen, getrocknet und eingedampft. Den Rückstand chromatographiert man über Kieselgel (Elution mit Methylenchlorid/Methanol 98 : 2). Aus dem Eluat erhält man schließlich 4,8 g = 62 % d. Th. der Titelverbindung vom Fp. 78-79 °C.

Der Ausgangsester wurde wie folgt erhalten :

a) 6,0 g (46 mMol) Itaconsäure und 7,7 g (46 mMol) 3,4-Dimethoxybenzylamin werden unter einer Stickstoffatmosphäre 2 Stunden auf 130 °C erhitzt. Nach Abkühlen auf ca. 100 °C gibt man unter mechanischem Rühren zu dem zähflüssigen Reaktionsgemisch 70 ml 10 %ige Natronlauge und kühlt anschließend auf Raumtemperatur. Zur Entfernung unlöslicher Anteile schüttelt man die Natriumsalzlösung mit 50-100 ml Essigester aus und säuert die wäßrige Phase mit verdünnter Salzsäure an. Das ausgefallene Öl wird in Methylenchlorid aufgenommen, die Methylenchloridphase getrocknet und eingeengt. Der Rückstand kristallisiert bei Ätherzugabe. Man erhält 8,7 g, 68 % 1-(3,4-Dimethoxybenzyl)-4-carboxy-pyrrolidin-2-on vom Fp. 176-178 °C.

b) 8,6 g (30 mMol) der Säure werden in 120 ml absolutem Äthanol gelöst und die Lösung 2 Stunden unter Rückfluß gekocht, während man ständig trocknes HCl-Gas einleitet. Anschließend wird der überschüssige Alkohol im Vakuum abdestilliert und der Rückstand mit 30 %iger Sodalösung unter Kühlung auf pH 8 gebracht. Nach Ausschütteln mit Methylenchlorid, Waschen, Trocknen und Abdampfen des Lösungsmittels erhält man ca. 10 g 1-(3,4-Dimethoxybenzyl)-4-äthoxycarbonyl-pyrrolidin-2-on als gelbes Öl, das roh weiter umgesetzt werden kann.

Der gemäß b) erhaltene Ester kann auch analog dem folgenden Verfahren hergestellt werden :

10 g (0,064 Mol) Itaconsäuredimethylester und 7,7 g (0,064 Mol) α-Phenyläthylamin werden unter Stickstoffatmosphäre 2 Stunden bei 120-130 °C gerührt. Das Reaktionsprodukt wird dann bei 150-155 °C und 0,1 Torr fraktioniert destilliert.

Man erhält 9,5 g = 61 % d. Th. eines hellen Öles, das unverändert zur selektiven Reduktion eingesetzt wurde.

Beispiel 2

1-(p-Fluorbenzyl)-4-hydroxymethyl-pyrrolidin-2-on

4,0 g (16 Mol) 1-(p-Fluorbenzyl)-4-äthoxycarbonyl-pyrrolidin-2-on werden in 60 ml tertiärem Butanol gelöst und mit 1,5 g Natriumborhydrid versetzt. Man bringt auf Rückflußtemperatur und fügt während 1 Stunde allmählich 12 ml trocknes Methanol hinzu.

Nach einer weiteren Stunde wird das Reaktionsgemisch im Vakuum eingedampft. Den Rückstand verdünnt man mit Wasser, schüttelt die Titelverbindung mit Methylenchlorid aus und arbeitet wie unter Beispiel 1 beschrieben auf. Die Ausbeute an reiner Titelverbindung beträgt 2,5 g vom Fp. 121-122 °C, das sind 72 % d. Th., bezogen auf den Rohester.

Der Ester wurde wie folgt erhalten :

7 g (54 mMol) Itaconsäure und 6,8 g (54 mMol) 4-Fluorbenzylamin werden zusammen mit 50 ml Wasser 3 Stunden unter Rückfluß gekocht. Anschließend macht man mit 10 %iger Natronlauge deutlich alkalisch, schüttelt die wasserunlöslichen Bestandteile mit Essigester aus und säuert die wäßrige Phase mit HCl an. Die weitere Aufarbeitung erfolgt analog Beispiel 1. Man erhält hellbraune Kristalle der entsprechenden Carbonsäure vom Fp. 152-153 °C, die gemäß dem in Beispiel 1 beschriebenen Verfahren verestert wird.

Nach dem oben beschriebenen Verfahren wurden die in der umseitigen Tabelle aufgeführten Endprodukte über die ebenfalls angegebenen Carbonsäuren erhalten :

(Siehe Tabellen Seite 6 ff.)

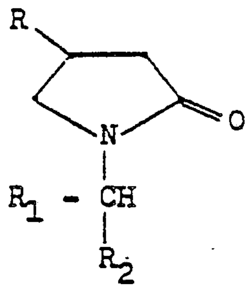

| R | $R_1$ | $R_2$ | Fp. °C | $Kp_{0,1}$ °C | R | Fp. °C |
|---|---|---|---|---|---|---|
| $-CH_2-OH$ | H | 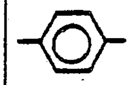 | 63-64 | | $CO_2H$ | 144-146 |
| $-CH_2-OH$ | H | 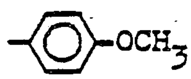 | | 188-190 | $CO_2H$ | 158-159 |
| $-CH_2-OH$ | H | 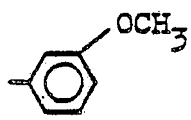 | | 165-168 | $CO_2H$ | 112-113 |
| $-CH_2-OH$ | H | 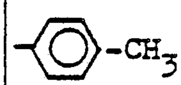 | | 165-168 | $CO_2H$ | 156-157 |
| $-CH_2-OH$ | H | 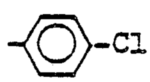 | 77-78 | 162-165 | $CO_2H$ | 160-161 |
| $-CH_2-OH$ | H | 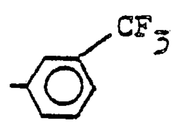 | | 95-96 | $CO_2H$ | - |
| $-CH_2-OH$ | $CH_3$ | 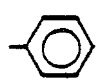 | | 146-147 | $CO_2H$ | 180-181 |
| $-CH_2-OH$ | $CH_3$ | 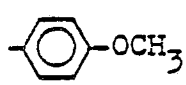 | | 170-172 | $CO_2H$ | 165-166 |
| $-CH_2-OH$ | H | 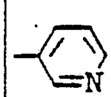 | | 178-182 | $CO_2H$ | - |

| R | $R_1$ | $R_2$ | Fp. °C | $Kp_{0,1}$ °C | R | Fp. °C |
|---|---|---|---|---|---|---|
| $-CH_2-OH$ | H | 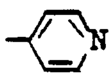 | 79-80 | | $CO_2H$ | |
| $-CH_2-OH$ | H | 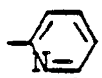 | | 160-163 | $CO_2H$ | |
| $-CH_2-OH$ | H | 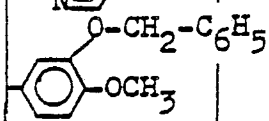 | Öl | | | |
| $-CH_2-OH$ | H | 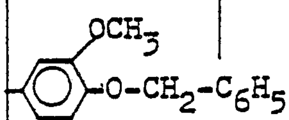 | Öl | | - | |
| $-CH_2-OH$ | H | 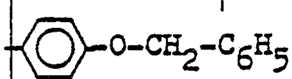 | 104-105 | | - | |
| $-CH_2-OH$ | H | 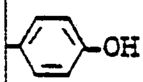 | 150-152 | | - | |
| $-CH_2-OH$ | H | 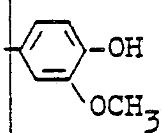 | 99-101 | | - | |
| $-CH_2-OH$ | H | 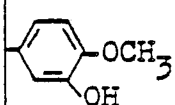 | 128 | | - | |
| $-CH_2-OH$ | H | 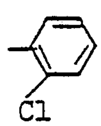 | 84-86 | | - | |
| | | | | | - | |

Pharmazeutische Formulierungsbeispiele

A) Tabletten

| | pro Tablette |
|---|---|
| Wirkstoff | 100 mg |
| Milchzucker (pulverisiert) | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B) Tabletten

| | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

C) Ampullen

a) Wirkstofflösung

| | |
|---|---|
| 1-Benzyl-4-hydroxymethyl-pyrrolidin-2-on | 100 mg |
| Polyäthylenglykol ad | 5 ml |

b) Diluens

| | |
|---|---|
| doppelt destilliertes Wasser | 5 ml |

Vor der Injektion ist das Diluens dem Inhalt der Wirkstoffampulle zuzufügen. Man erhält 10 ml einer spritzfertigen Lösung, enthaltend 100 mg Wirkstoff.

D) Ampullen

a) Wirkstofflösung

| | |
|---|---|
| 1-(4-Methoxybenzyl)-4-hydroxymethyl-pyrrolidin-2-on | 120,0 mg |
| Glycofurol ad | 3,5 ml |

b) Diluens

| | |
|---|---|
| Natriumchlorid | 67,5 mg |
| doppelt destilliertes Wasser ad | 6,5 ml |

Vor der Injektion ist das Diluens dem Inhalt der Wirkstoffampulle zuzufügen. Man erhält 10 ml einer spritzfertigen Lösung, enthaltend 120 mg Wirkstoff.

E) Ampullen

| | |
|---|---|
| 1-Pyridyl-(4)-methyl-4-hydroxymethyl-pyrrolidon-2-on | 80 mg |

| | |
|---|---|
| Natriumpyrosulfit | 20 mg |
| Dinatriumsalz der Äthylendiamintetraessigsäure | 8 mg |
| Natriumchlorid | 50 mg |
| doppelt destilliertes Wasser ad | 1 000 mg |

Herstellung

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 80 mg Wirkstoff.

Die neuen Verbindungen sollen in einer Dosierung von 50-200, vorzugsweise 70-150 mg/Dosis angewendet werden.

**Patentansprüche**

1. In 1-Stellung substituierte 4-Hydroxymethyl-pyrrolidinone der allgemeinen Formel

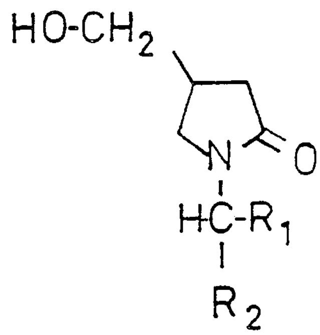

in der

$R_1$ Wasserstoff oder Methyl

$R_2$ einen gegebenenfalls durch Alkyl mit 1-2 Kohlenstoffatomen, Alkoxy mit 1-2 Kohlenstoffatomen, Fluor, Chlor, Brom, Trifluormethyl, Nitro, Benzyloxy oder Hydroxy ein-, zwei- oder dreifach substituierten Phenylrest oder einen Pyridylrest bedeutet.

2. In 1-Stellung substituierte 4-Hydroxymethyl-pyrrolidinone gemäß Anspruch 1, worin $R_1$ Wasserstoff und $R_2$ einen gegebenenfalls ein-, zwei- oder dreifach durch Methoxy substituierten Phenylrest bedeutet.

3. 1-Benzyl-4-hydroxymethyl-pyrrolidin-2-on.

4. 1-(4-Methoxybenzyl)-4-hydroxymethyl-pyrrolidin-2-on.

5. 1-(3,4-Dimethoxybenzyl)-4-hydroxymethyl-pyrrolidin-2-on.

6. Verfahren zur Herstellung von 1-Stellung substituierten 4-Hydroxymethyl-pyrrolidinonen der allgemeinen Formel

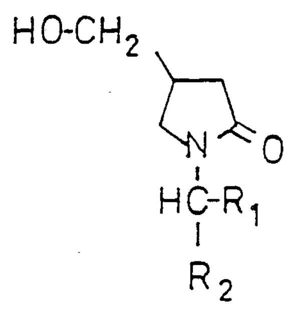

(I)

in der

$R_1$ Wasserstoff oder Methyl und

$R_2$ einen gegebenenfalls durch Alkyl mit 1-2 Kohlenstoffatomen, Alkoxy mit 1-2 Kohlenstoffatomen, Fluor, Chlor, Brom, Trifluormethyl, Nitro, Benzyloxy oder Hydroxy ein-, zwei- oder dreifach substituierten Phenylrest oder einen Pyridylrest bedeuten, dadurch gekennzeichnet, daß man

a) einen Pyrrolidinon-4 carbonsäureester der allgemeinen Formel

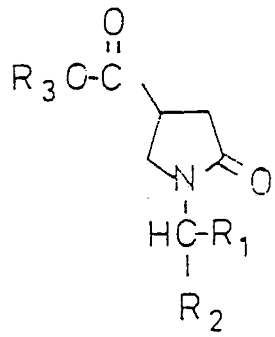

(IIa)

in der

$R_1$ und $R_2$ die oben angeführte Bedeutung besitzen und $R_3$ eine Alkylgruppe mit 1 bis 2 Kohlenstoffatomen bedeutet, mit einem komplexen Alkali-Borhydrid selektiv reduziert.

7. Verfahren zur Herstellung von Verbindungen der allgemeinen Formel I

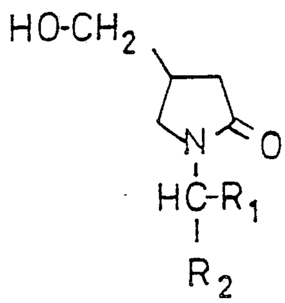

(I)

worin

$R_1$ Wasserstoff oder Methyl und

$R_2$ einen hydroxysubstituierten Phenylrest bedeutet,

dadurch gekennzeichnet, daß man eine Verbindung der allgemeinen Formel I, worin $R_2$ einen entsprechenden benzyloxysubstituierten Phenylrest bedeutet, katalytisch hydriert.

8. Verfahren zur Herstellung von Verbindungen der allgemeinen Formel I

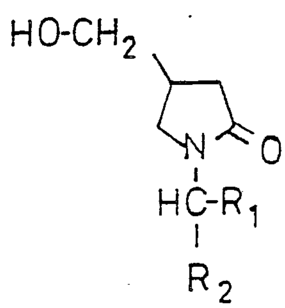

(I)

in der

$R_1$ Wasserstoff oder Methyl und

$R_2$ einen gegebenenfalls durch Alkyl mit 1-2 Kohlenstoffatomen, Alkoxy mit 1-2 Kohlenstoffatomen, Fluor, Chlor, Brom, Trifluormethyl, Nitro, Benzyloxy oder Hydroxy ein-, zwei- oder dreifach substituierten Phenylrest

dadurch gekennzeichnet, daß man Itaconsäuredimethyl — oder diethylester mit dem entsprechenden Amin

$$H_2N—CHR_1R_2$$

worin $R_1$ und $R_2$ die zuvor genannte Bedeutung aufweisen, umsetzt und mit einem komplexen Alkali-Borhydrid selektiv reduziert.

9. Pharmazeutische Zusammensetzung, enthaltend als Wirkstoff eine oder mehrere Verbindungen der allgemeinen Formel I in Kombination mit üblichen Hilfs und/oder Trägerstoffen.

10. Verbindungen der allgemeinen Formel

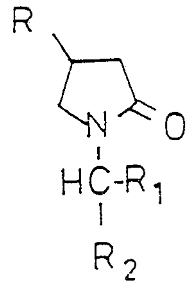

(II)

$R_1$ Wasserstoff oder Methyl,

$R_2$ einen durch Alkyl mit 1-2 Kohlenstoffatomen, Alkoxy mit 1-2 Kohlenstoffatomen, Fluor, Chlor, Brom, Trifluormethyl, Nitro, Benzyloxy oder Hydroxy ein-, zwei- oder dreifach substituierten Phenylrest oder einen Pyridylrest und

R eine der Gruppen- COOH und —COO—$R_3$ mit $R_3$ = Alkyl mit 1 bis 2 Kohlenstoffatomen bedeutet.

**Claims**

1. 4-Hydroxymethylpyrrolidinones of general formula

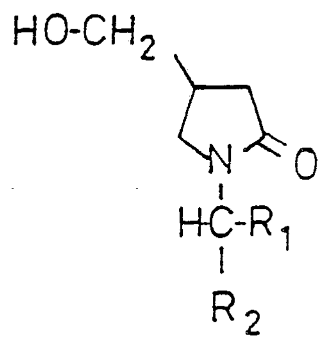

substituted in the 1-position, wherein

$R_1$ represents hydrogen or methyl

$R_2$ represents a phenyl group optionally mono-di or tri-substituted by alkyl with 1 to 2 carbon atoms, alkoxy with 1 to 2 carbon atoms, fluorine, chlorine, bromine, trifluoromethyl, nitro, benzyloxy or hydroxy ; or a pyridyl group.

2. 4-Hydroxymethyl-pyrrolidinones substituted in the 1-position according to claim 1, wherein $R_1$ represents hydrogen and $R_2$ represents a phenyl group optionally mono-, di- or tri-substituted by methoxy.

3. 1-Benzyl-4-hydroxymethyl-pyrrolidin-2-one.

4. 1-(4-Methoxybenzyl)-4-hydroxymethyl-pyrrolidin-2-one.

5. 1-(3,4-Dimethoxybenzyl)-4-hydroxymethyl-pyrrolidin-2-one.

6. Process for preparing 4-hydroxymethyl-pyrrolidinones substituted in the 1-position of general formula

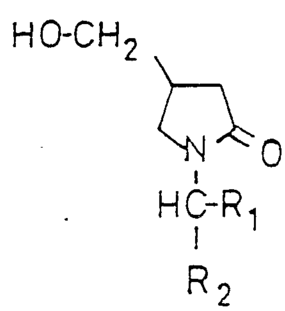

(I)

wherein

$R_1$ represents hydrogen or methyl and

$R_2$ represents a phenyl group optionally mono-, di- or tri-substituted by alkyl with 1 to 2 carbon atoms, alkoxy with 1 to 2 carbon atoms, fluorine, chlorine, bromine, trifluoromethyl, nitro, benzyloxy or hydroxy, or a pyridyl group, characterised in that

a) a pyrrolidinone-4-carboxylic acid ester of general formula

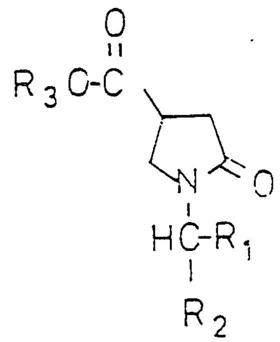

(IIa)

wherein

$R_1$ and $R_2$ are defined as above and $R_3$ represents and alkyl group with 1 to 2 carbon atoms, is selectively reduced with a complex alkali metal borohydride.

7. Process for preparing compounds of general formula I

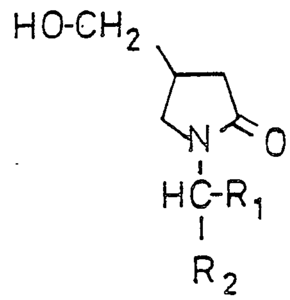

(I)

wherein

$R_1$ represents hydrogen or methyl and

$R_2$ represents a hydroxy-substituted phenyl group, characterised in that a compound of general formula I wherein $R_2$ represents a corresponding benzyloxy-substituted phenyl group is catalytically hydrogenated.

8. Process for preparing compounds of general formula I

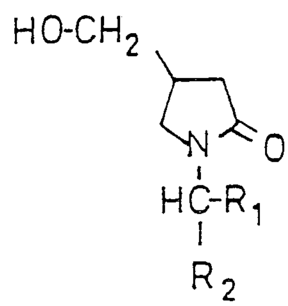

(I)

wherein

$R_1$ represents hydrogen or methyl and

$R_2$ represents a phenyl group optionally mono-, di- or tri-substituted by alkyl with 1 to 2 carbon atoms, alkoxy with 1 to 2 carbon atoms, fluorine, chlorine, bromine, trifluoromethyl, nitro, benzyloxy or hydroxy,

characterised in that dimethyl or diethyl itaconate is reacted with the corresponding amine

$$H_2N—CHR_1R_2$$

wherein $R_1$ and $R_2$ are defined as above, and is selectively reduced with a complex alkali metal borohydride.

9. Pharmaceutical composition containing as active substance one or more compounds of general formula I in conjunction with conventional excipients and/or carriers.

10. Compounds of general formula

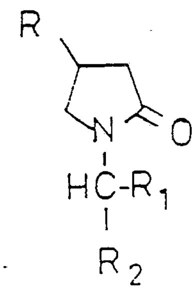

(II)

wherein

$R_1$ represents hydrogen or methyl,

$R_2$ represents a phenyl group mono-, di- or tri-substituted by alkyl with 1 to 2 carbon atoms, alkoxy with one to two carbon atoms, fluorine, chlorine, bromine, trifluoromethyl, nitro, benzyloxy or hydroxy, or a pyridyl group and

R represents one of the groups COOH and $—COO—R_3$ wherein $R_3$ = alkyl with 1 to 2 carbon atoms.

**Revendications**

1. 4-hydroxyméthyl-pyrrolidinones substituées en position 4 répondant à la formule générale

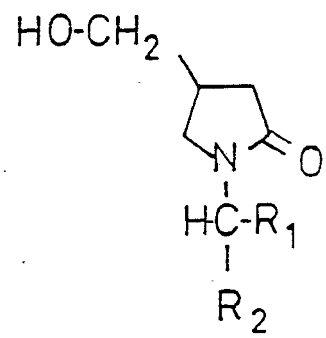

dans laquelle

$R_1$ désigne un atome d'hydrogène ou un groupe méthyle

$R_2$ désigne un radical phényle substitué une fois, deux fois ou trois fois par un groupe alkyle en $C_1$ à $C_2$, un groupe alcoxy en $C_1$ à $C_2$, un atome de fluor, de chlore, de brome, un groupe trifluorométhyle, nitro, benzyloxy ou hydroxy, ou un radical pyridyle.

2. 4-hydroxyméthyl-pyrrolidinones substituées en position 1 suivant la revendication 1, dans lesquelles $R_1$ désigne un atome d'hydrogène et $R_2$ désigne un radical phényle substitué le cas échéant une fois, deux fois ou trois fois par un groupe méthoxy.

3. 1-benzyl-4-hydroxyméthyl-pyrrolidin-2-one.

4. 1-(4-méthoxybenzyl)-4-hydroxyméthyl-pyrrolidin-2-one.

5. 1-(3,4-diméthoxybenzyl)-4-hydroxyméthyl-pyrrolidin-2-one.

6. Procédé de préparation de 4-hydroxyméthyl-pyrrolidinones substituées en position 1, répondant à la formule générale

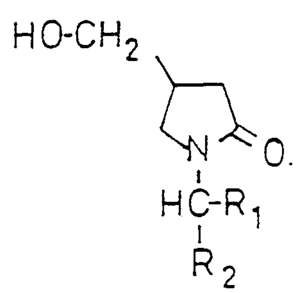

(I)

dans laquelle

$R_1$ désigne un atome d'hydrogène ou un groupe méthyle, et

$R_2$ désigne un radical phényle substitué le cas échéant une fois, deux fois ou trois fois par un groupe alkyle en $C_1$ à $C_2$, alcoxy en $C_1$ à $C_2$, par un atome de fluor, de chlore, de brome, par un groupe trifluorométhyle, nitro, benzyloxy ou hydroxy, ou un radical pyridyle, caractérisé en ce que on réduit sélectivement un ester pyrrolidinone-4-carboxylique répondant à la formule générale

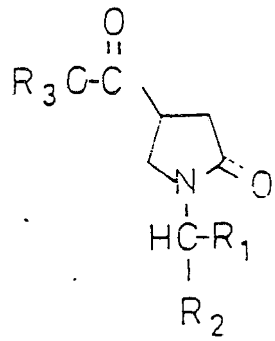

(IIa)

dans laquelle

$R_1$ et $R_2$ ont la signification indiquée ci-dessus et $R_3$ désigne un groupe alkyle en $C_1$ à $C_2$, par un borohydrure alcalin complexe.

7. Procédé de préparation de composés répondant à la formule générale I

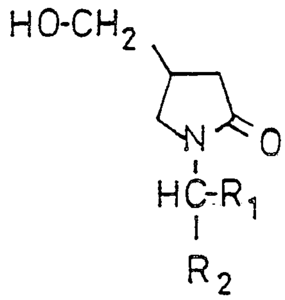

(I)

dans laquelle

$R_1$ désigne un atome d'hydrogène ou un groupe méthyle, et

$R_2$ désigne un radical phényle substitué par un groupe hydroxy, caractérisé en ce que on hydrogène catalytiquement un composé répondant à la formule générale I, dans laquelle $R_2$ désigne un radical phényle substitué par un groupe benzyloxy approprié.

8. Procédé de préparation de composés répondant à la formule générale I

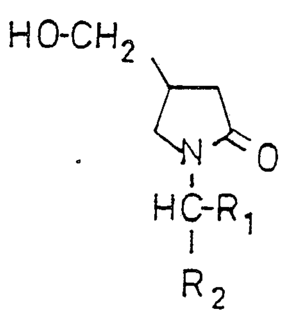

(I)

dans laquelle

$R_1$ désigne un atome d'hydrogène ou un groupe méthyle, et

$R_2$ désigne un radical phényle substitué le cas échéant, une fois, deux fois ou trois fois par un groupe alkyle en $C_1$ à $C_2$, un groupe alcoxy en $C_1$ à $C_2$, un atome de fluor, de chlore, de brome, un groupe trifluorométhyle, nitro, benzyloxy ou hydroxy,

caractérisé en ce qu'on fait réagir un ester diméthylique ou diéthylique de l'acide itaconique avec l'amine appropriée

$H_2N—CHR_1R_2$

dans laquelle $R_1$ et $R_2$ présentent la signification précédemment indiquée, et on le réduit sélectivement avec un borohydrure alcalin complexe.

9. Composition pharmaceutique contenant comme substance active un ou plusieurs composés répondant à la formule générale I en association avec des adjuvants et/ou supports habituels.

10. Composés répondant à la formule générale

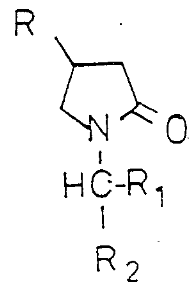

(II)

dans laquelle

$R_1$ désigne l'hydrogène ou un groupe méthyle,

$R_2$ désigne un radical phényle substitué une fois, deux fois ou trois fois par un groupe alkyle en $C_1$ à $C_2$, alcoxy en $C_1$ à $C_2$, un atome de fluor, de chlore, de brome, un groupe trifluorométhyle, nitro, benzyloxy ou hydroxy ou un radical pyridyle, et

R désigne un des groupes COOH et —COO— dans lequel $R_3$ est un groupe alkyle en $C_1$ à $C_2$.